# EUROPEAN PATENT APPLICATION

(11) **EP 3 124 012 A1**
(43) Date of publication of application: **01.02.2017**
(21) Application number: 15769659.2
(22) Date of filing: 20.02.2015
(51) Int. Cl.: A61K 8/41, A61K 8/20, A61K 8/26, A61K 8/27, A61K 8/34, A61K 8/368, A61K 8/37, A61K 8/73, A61Q 15/00

(54) **DEODORANT COMPOSITION AND DEODORANT AGENT**

(30) Priority: 26.03.2014 JP 2014063891
(71) Applicant: Mandom Corporation, Osaka-shi, Osaka 540-8530 (JP)
(72) Inventor: KASAHARA, Keiji, Osaka-shi Osaka 540-8530 (JP); HISAHARA, Takeshi, Osaka-shi Osaka 540-8530 (JP)
(74) Representative: TBK
(86) International application number: PCT/JP2015/054802
(87) International publication number: WO 2015/146398

(57) **Abstract**

Provided are a deodorant composition that can dramatically improve the remaining performance of a bactericide on an application object after application and a deodorant product comprising the deodorant composition. The deodorant composition according to the present invention comprises ethanol, a bactericide, a hydrophobized hydroxyalkyl cellulose, and an ester compound having a molecular weight of 150 or more; and the deodorant product according to the present invention comprises a container and the deodorant composition filled in the container.

## Description

### Technical Field

The present invention relates to a deodorant composition containing a bactericide. The present invention also relates to a deodorant product comprising the deodorant composition filled in a container.

### Background Art

Hircismus, which makes people uncomfortable, is generated by decomposition of a mixture of sweat and sebum by skin normal florae. In order to suppress the uncomfortable hircismus, deodorant compositions have been used. Such a deodorant composition is filled in a container and is used as a deodorant product. The deodorant products are used in various forms such as gel, cream, lotion, aerosol spray, mist, and stick forms.

Specifically, a roll-on deodorant product eliminates the necessity of once applying a composition onto a hand and then applying the composition onto an application object, enables direct and uniform application of the composition onto the application object, and thus is excellent in usability. In addition, the roll-on deodorant product contains a content having high adhesive properties, thus is likely to provide higher hircismus suppressive effect than that of aerosol deodorant products, and is preferred. On this account, the utilization ratio of the roll-on deodorant product has increased among the deodorant products in recent years.

As an example of the deodorant composition, Patent Document 1 discloses a deodorant composition comprising a hydroxyalkylated cyclodextrin, an active component for antiperspirant, and water. Patent Document 1 describes that a deodorant composition having novel deodorization effect and durability over a long time can be provided.

Patent Document 2 discloses a deodorant composition comprising an antiperspirant and/or a bactericide (A), an unsaturated fatty acid (B), an antioxidant (C), and an aqueous medium (D). Patent Document 2 describes that a deodorant product that has excellent quick-drying properties on the skin, is easy to handle, and causes a low irritation feeling can be provided.

Patent Document 3 discloses a deodorant composition comprising 0.05 to 5.0% by weight of methacrylate chloride-choline ester copolymer (A), 10.0 to 50.0% by weight of ethanol (B), and 0.5 to 3.0% by weight of zinc sulfocarbolate and/or 0.5 to 10.0% by weight of aluminum hydroxychloride (C). Patent Document 3 describes that a deodorant effect with less irritation and good feeling is achieved.

### Citation List

### Patent Literature

Patent Document 1: JP-A No. 2003-95906
Patent Document 2: JP-A No. 2006-76997
Patent Document 3: JP-A No. 2002-370958

### Summary of Invention

### Technical Problem

However, when such an existing deodorant composition as described in Patent Document 1 to 3 are filled in a container and a resulting deodorant product is used, a sufficient amount of a bactericide is difficult to remain on an application object such as the underarm after application. On this account, the bactericidal effect is insufficiently achieved over time after the application, and the deodorization effect is unsatisfactory achieved unfortunately.

To address the problem, the present invention has an object to provide a deodorant composition that can dramatically improve the remaining performance of a bactericide on an application object after application and also has another object to provide a deodorant product comprising the deodorant composition.

### Solution to Problem

The present invention provides a deodorant composition comprising ethanol, a bactericide, a hydrophobized hydroxyalkyl cellulose, and an ester compound having a molecular weight of 150 or more.

It is preferable that the ester compound have a logP value of not less than 1 and not more than 40.

It is preferable that the ester compound be an ester compound selected from the group consisting of isononyl isononanoate, glyceryl tri-2-ethylhexanoate, diisopropyl adipate, diisobutyl adipate, polypropylene glycol-3 benzyl ether myristate, isopropyl myristate, propylene glycol isostearate, isopropyl palmitate, 2-ethylhexyl palmitate, cetyl 2-ethylhexanoate, pentaerythrityl tetraoctanoate, octyldodecyl myristate, and pentaerythrityl tetraisostearate.

It is preferable that the bactericide be a bactericide selected from the group consisting of isopropylmethylphenol, benzalkonium chloride, hinokitiol, triclosan, and salicylic acid.

It is preferable that the deodorant composition according to the present invention comprise a nonionic surfactant.

It is preferable that the deodorant composition according to the present invention comprise an antiperspirant. It is preferable that the antiperspirant be an antiperspirant selected from the group consisting of aluminum hydroxychloride, zinc p-phenolsulfonate, and alums.

The present invention also provides a deodorant product comprising a container and the deodorant composition, and the deodorant composition is filled in the container.

It is preferable that the deodorant product according to the present invention be a non-aerosol preparation.

### Advantageous Effects of Invention

A deodorant composition according to the present invention comprises ethanol, a bactericide, a hydrophobized hydroxyalkyl cellulose, and an ester compound having a molecular weight of 150 or more. The deodorant composition according to the present invention comprises the hydrophobized hydroxyalkyl cellulose and the ester compound having a molecular weight of 150 or more in combination, and thus can dramatically improve the remaining performance of the bactericide on an application object after application.

### Description of Embodiments

The present invention will now be described in detail.

The deodorant composition according to the present invention at least comprises ethanol, a bactericide, a hydrophobized hydroxyalkyl cellulose, and an ester compound having a molecular weight of 150 or more.

In the present specification, the ethanol is also called "ethanol (A)" or "component (A)"; the bactericide is also called "bactericide (B)" or "component (B)"; the hydrophobized hydroxyalkyl cellulose is also called "hydrophobized hydroxyalkyl cellulose (C)" or "component (C)"; and the ester compound having a molecular weight of 150 or more is also called "ester compound (D) having a molecular weight of 150 or more", "ester compound (D)", or "component (D)".

In the present invention, the hydrophobized hydroxyalkyl cellulose (C) and the ester compound (D) having a molecular weight of 150 or more are used in combination as the components used in combination with the ethanol (A) and the bactericide (B), and thus the remaining performance of the bactericide (B) on an application object such as the underarm can be dramatically improved. On this account, the bactericidal effect can be sufficiently achieved even over time after the application.

In order to effectively improve the remaining performance of the bactericide after application and the durability of the bactericidal effect after application, the deodorant composition according to the present invention preferably comprises a nonionic surfactant.

In order to improve the antiperspirant effect, the deodorant composition according to the present invention may comprise an antiperspirant. This can further improve the effect as the deodorant composition.

In the present specification, the nonionic surfactant is also called "nonionic surfactant (E)" or "component (E)"; and the antiperspirant is also called "antiperspirant (F)" or "component (F)".

In order to more effectively achieve the effect of the component (C), the deodorant composition according to the present invention preferably comprises water. The deodorant composition according to the present invention may contain other components in addition to the component (A) to the component (F) and water.

As each component contained in the deodorant composition according to the present invention, that is, as each of the components (A) to (F), water, and the other components, a single component may be used, or two or more components may be used.

Each component used or suitably used in the deodorant composition according to the present invention will next be described in detail.

### (Ethanol (A))

In the deodorant composition according to the present invention, the ethanol (A) can be contained in any amounts. Relative to 100% by mass of the deodorant composition, the amount of the ethanol (A) is preferably 20.0% by mass or more, more preferably 40.0% by mass or more, even more preferably more than 50.0% by mass, and particularly preferably 60.0% by mass or more and is preferably 99.5% by mass or less, more preferably 95.5% by mass or less, even more preferably 90.0% by mass or less, particularly preferably 89.0% by mass or less, and most preferably 80.0% by mass or less. If containing the ethanol (A) at the above lower limit or more, the deodorant composition has higher usability and quick-drying properties. The upper limit of the amount of the ethanol (A) can be appropriately adjusted depending on the amounts of the other components.

### (Bactericide (B))

The bactericide (B) contained in the deodorant composition according to the present invention has a bactericidal action. In the present invention, the remaining performance of the bactericide after application and the durability of the bactericidal effect after application are considerably excellent.

Examples of the bactericide (B) include isopropylmethylphenol, benzalkonium chloride, hinokitiol, triclosan, and salicylic acid. As the bactericide (B), a single bactericide may be used, or two or more bactericides may be used.

In order to effectively improve the remaining performance of the bactericide (B) after application and the durability of the bactericidal effect after application, the bactericide (B) is preferably a bactericide (at least one bactericide) selected from the group consisting of isopropylmethylphenol, benzalkonium chloride, hinokitiol, triclosan, and salicylic acid and is more preferably a bactericide (at least one bactericide) selected from the group consisting of isopropylmethylphenol, benzalkonium chloride, and hinokitiol.

In the deodorant composition according to the present invention, the bactericide (B) can be contained in any amounts. Relative to 100% by mass of the deodorant composition, the amount of the bactericide (B) is preferably 0.01% by mass or more and more preferably 0.05% by mass or more and is preferably 2.0% by mass or less, more preferably 1.0% by mass or less, and even more preferably 0.5% by mass or less. If the bactericide (B) is contained at the above lower limit or more, the bactericidal effect is more effectively achieved. In order to improve the safety, the bactericide (B) is preferably contained at the above upper limit or less.

### (Hydrophobized hydroxyalkyl cellulose (C))

The hydrophobized hydroxyalkyl cellulose (C) contained the deodorant composition according to the present invention is a hydrophobized product of a hydroxyalkyl cellulose. The hydrophobized hydroxyalkyl cellulose (C) may be any hydrophobized hydroxyalkyl celluloses. Examples of the hydrophobized hydroxyalkyl cellulose (C) include hydrophobized hydroxypropyl methylcelluloses and hydrophobized hydroxyethylcelluloses. As the hydrophobized hydroxyalkyl cellulose (C), a single hydrophobized hydroxyalkyl cellulose may be used, or two or more hydrophobized hydroxyalkyl celluloses may be used.

The hydrophobized hydroxyalkyl cellulose (C) is preferably obtained by introduction of an alkyl group to a hydroxyalkyl cellulose. The hydrophobized hydroxypropyl methylcellulose is preferably obtained by introduction of an alkyl group to hydroxypropyl methylcellulose. The alkyl group is preferably a long-chain alkyl group, which has high hydrophobicity. The hydrophobized hydroxyalkyl cellulose (C) is preferably obtained by introduction of an alkyl group (alkyl group imparting hydrophobicity) having 12 or more carbon atoms, which has high hydrophobicity, to a hydroxyalkyl cellulose.

The hydrophobized hydroxyalkyl cellulose (C) preferably has an alkyl group (alkyl group imparting hydrophobicity) having 12 or more carbon atoms. The upper limit of the number of carbon atoms of the alkyl group imparting hydrophobicity is not limited to particular values. The number of carbon atoms of the alkyl group imparting hydrophobicity is preferably 22 or less. The alkyl group imparting hydrophobicity may be a linear group or a branched group. The number of carbon atoms of the linear moiety of the alkyl group imparting hydrophobicity is preferably 12 or more.

The alkyl group imparting hydrophobicity is exemplified by a dodecyl group, a tridecyl group, a tetradecyl group, a pentadecyl group, a hexadecyl group, a heptadecyl group, an octadecyl group, a nonadecyl group, an eicosyl group, a heneicosyl group, and a docosyl group.

The hydrophobized hydroxyalkyl cellulose (C) is preferably a hydroxyalkyl cellulose alkyloxy ether. The hydrophobized hydroxypropyl methylcellulose is preferably a hydroxypropyl methylcellulose alkyloxy ether. The alkyloxy group of the hydroxyalkyl cellulose alkyloxy ether preferably has 12 or more carbon atoms and preferably has 22 or less carbon atoms. The hydrophobized hydroxyalkyl cellulose (C) may have a group including an alkyl group imparting hydrophobicity (such as long-chain alkyl groups) that is not the alkyloxy group.

The alkyloxy group is exemplified by a dodecyloxy group (lauroxy group), a tridecyloxy group, a tetradecyloxy group (myristyloxy group), a pentadecyloxy group, a hexadecyloxy group (cetyloxy group), a heptadecyloxy group, an octadecyloxy group (stearoxy group), a nonadecyloxy group, an eicosyloxy group, a heneicosyloxy group, and a docosyloxy group.

Examples of the hydrophobized hydroxyalkyl cellulose (C) include hydroxyalkyl cellulose dodecyloxy ethers (hydroxyalkyl cellulose lauroxy ethers), hydroxyalkyl cellulose tridecyloxy ethers, hydroxyalkyl cellulose tetradecyloxy ethers (hydroxyalkyl cellulose myristyloxy ethers), hydroxyalkyl cellulose pentadecyloxy ethers, hydroxyalkyl cellulose hexadecyloxy ethers (hydroxyalkyl cellulose cetyloxy ethers), hydroxyalkyl cellulose heptadecyloxy ethers, hydroxyalkyl cellulose octadecyloxy ethers (hydroxyalkyl cellulose stearoxy ethers), hydroxyalkyl cellulose nonadecyloxy ethers, hydroxyalkyl cellulose eicosyloxy ethers, hydroxyalkyl cellulose heneicosyloxy ethers, and hydroxyalkyl cellulose docosyloxy ethers.

The hydrophobized hydroxyalkyl cellulose (C) is particularly preferably exemplified by hydroxyalkyl cellulose octadecyloxy ethers (hydroxyalkyl cellulose stearoxy ethers) and most preferably exemplified by hydroxypropyl methylcellulose octadecyloxy ether (hydroxypropyl methylcellulose stearoxy ether).

As the hydrophobized hydroxyalkyl cellulose (C), a commercial product can be used. The commercial product of the hydrophobized hydroxyalkyl cellulose (C) is exemplified by SANGELOSE 60L, SANGELOSE 60M, SANGELOSE 90L, and SANGELOSE 90M (trade name; hydroxypropyl methylcellulose stearoxy ethers, manufactured by Daido Chemical Corporation).

In the deodorant composition according to the present invention, the hydrophobized hydroxyalkyl cellulose (C) can be contained in any amounts. Relative to 100% by mass of the deodorant composition, the amount of the hydrophobized hydroxyalkyl cellulose (C) is preferably 0.3% by mass or more and more preferably 0.5% by mass or more and is preferably 5.0% by mass or less and more preferably 3.0% by mass or less. If the hydrophobized hydroxyalkyl cellulose (C) is contained at the above lower limit or more, the remaining performance of the bactericide after application and the durability of the bactericidal effect after application are more effectively improved. If containing the hydrophobized hydroxyalkyl cellulose (C) at the above upper limit or less, the adhesiveness of the coating to the skin after the application and drying of the deodorant composition according to the present invention is much improved, and thus the durability of the bactericidal effect after application and the feeling of use are much improved.

### (Ester compound having a molecular weight of 150 or more (D))

The ester compound (D) contained in the deodorant composition according to the present invention has a molecular weight of 150 or more. When the molecular weight of the ester compound (D) is the above lower limit or more, the remaining performance of the bactericide after application and the durability of the bactericidal effect after application are effectively improved. As the ester compound (D), a single compound may be used, or two or more compounds may be used.

In order to prevent the composition from running off due to sweat and to effectively improve the remaining performance of the bactericide after application and the durability of the bactericidal effect after application, the ester compound (D) preferably has a logP value of 40 or less, more preferably 20 or less, and even more preferably 10 or less. The ester compound (D) preferably has a logP value of 1 or more and more preferably 2 or more. The logP value is a value calculated based on the database, "ChemSpider".

In order to more effectively improve the remaining performance of the bactericide after application and the durability of the bactericidal effect after application, the ester compound (D) is preferably an ester compound (at least one ester compound) selected from the group consisting of isononyl isononanoate, glyceryl tri-2-ethylhexanoate, diisopropyl adipate, diisobutyl adipate, polypropylene glycol-3 benzyl ether myristate, isopropyl myristate, propylene glycol isostearate, isopropyl palmitate, 2-ethylhexyl palmitate, cetyl 2-ethylhexanoate, pentaerythrityl tetraoctanoate, octyldodecyl myristate, and pentaerythrityl tetraisostearate.

As the ester compound (D), a commercial product can be used.

In order to further effectively improve the remaining performance of the bactericide after application and the durability of the bactericidal effect after application, the ester compound (D) is preferably an ester compound (at least one ester compound) selected from the group consisting of isononyl isononanoate, glyceryl tri-2-ethylhexanoate, diisopropyl adipate, polypropylene glycol-3 benzyl ether myristate, isopropyl myristate, propylene glycol isostearate, and isopropyl palmitate.

In order to effectively improve the remaining performance of the bactericide after application and the durability of the bactericidal effect after application, the ester compound (D) preferably has a molecular weight of 180 or more and more preferably 200 or more and preferably has a molecular weight of 5,000 or less, more preferably 2,000 or less, and even more preferably 600 or less.

In the deodorant composition according to the present invention, the ester compound (D) can be contained in any amounts. Relative to 100% by mass of the deodorant composition, the amount of the ester compound (D) is preferably 0.1% by mass or more and more preferably 0.3% by mass or more and is preferably 10.0% by mass or less and more preferably 5.0% by mass or less. If the ester compound (D) is contained at the above lower limit or more, the remaining performance of the bactericide after application and the durability of the bactericidal effect after application are more effectively improved. If the ester compound (D) is contained at the above upper limit or less, the usability and the quick-drying properties are much improved.

### (Nonionic surfactant (E))

The deodorant composition according to the present invention preferably comprises a nonionic surfactant (E). The nonionic surfactant (E) also works as a solubilizer for the ester compound (D) and functions to further improve the addition effect of the ester compound (D).

The nonionic surfactant (E) is not limited to particular surfactants and is exemplified by sorbitan fatty acid ester, glycerol fatty acid ester, polyglycerol fatty acid ester, polyoxyalkylene alkyl ether, polyoxyalkylene fatty acid ester, polyoxyalkylene sorbitan fatty acid ester, polyoxyalkylene castor oil, polyoxyalkylene hydrogenated castor oil, sucrose fatty acid ester, alkyl polyglucoside, and polyoxyethylene lanolin. As the nonionic surfactant (E), a single surfactant may be used, or two or more surfactants may be used.

In the deodorant composition according to the present invention, the nonionic surfactant (E) can be contained in any amounts. Relative to 100% by mass of the deodorant composition, the amount of the nonionic surfactant (E) is preferably 0.01% by mass or more and more preferably 0.1% by mass or more and is preferably 10.0% by mass or less and more preferably 5.0% by mass or less. If the nonionic surfactant (E) is contained at the above lower limit or more, the remaining performance of the bactericide after application and the durability of the bactericidal effect after application are more effectively improved. If the nonionic surfactant (E) is contained at the above upper limit or less, the usability and the quick-drying properties are much improved.

### (Antiperspirant (F))

The deodorant composition according to the present invention may contain an antiperspirant (F). The antiperspirant (F), for example, constricts the skin to suppress the generation of sweat. The antiperspirant (F) is not limited to particular agents. The antiperspirant (F) is exemplified by conventionally known antiperspirants. As the antiperspirant (F), a single antiperspirant may be used, or two or more antiperspirants may be used.

Examples of the antiperspirant (F) include aluminum salts and zinc p-phenolsulfonate. Examples of the aluminum salt include aluminum chloride, aluminum sulfate, aluminum potassium sulfate (potassium alum), aluminum ammonium sulfate (ammonium alum), aluminum acetate, aluminum hydroxychloride, and aluminum chlorohydroxy allantoinate. Antiperspirants other than these salts may be used.

In order to further improve the antiperspirant effect, the antiperspirant (F) is preferably at least one of aluminum salts and zinc p-phenolsulfonate, more preferably an antiperspirant (at least one antiperspirant) selected from the group consisting of aluminum hydroxychloride, zinc p-phenolsulfonate, and alums, and even more preferably an antiperspirant (at least one antiperspirant) selected from the group consisting of aluminum hydroxychloride, zinc p-phenolsulfonate, and aluminum potassium sulfate.

In the deodorant composition according to the present invention, the antiperspirant (F) can be contained in any amounts. Relative to 100% by mass of the deodorant composition, the amount of the antiperspirant (F) is preferably 0.01% by mass or more and more preferably 0.1% by mass or more and is preferably 25.0% by mass or less and more preferably 20.0% by mass or less in order to improve the antiperspirant effect, the feeling of use, and the safety.

### (Water)

The deodorant composition according to the present invention preferably contains water. The water is not limited to particular waters and is preferably purified water. When the deodorant composition contains water, the hydrophobized hydroxyalkyl cellulose (C) swells to further likely to achieve the effect.

Relative to 100% by mass of the deodorant composition, the amount of water is preferably 5.0% by mass or more and more preferably 10.0% by mass or more and is preferably 45.0% by mass or less and more preferably 30.0% by mass or less.

### (Other components)

The deodorant composition according to the present invention can contain a thickener. The thickener is exemplified by water-soluble polymers. Specific examples of the thickener include hydroxyalkyl celluloses. Examples of the hydroxyalkyl cellulose include hydroxymethylcellulose, hydroxyethylcellulose, hydroxypropylcellulose, and hydroxypropyl methylcellulose. As the thickener, a single thickener may be used, or two or more thickeners may be used.

To the deodorant composition according to the present invention, components commonly used in deodorant products or cosmetics can be added in addition to the above components. The deodorant composition according to the present invention can contain surfactants other than the nonionic surfactant (E), moisturizers, deodorizers, oil components, sequestrants, antiseptics, algefacients, anti-inflammatory agents, plant extracts, and perfumes, for example.

The surfactants other than the nonionic surfactant (E) are exemplified by anionic surfactants, amphoteric surfactants, and cationic surfactants. The moisturizer is exemplified by sorbitol. Glycerin is also usable as the moisturizer. The oil component is exemplified by hydrocarbons and silicones.

### (Other details of deodorant composition and deodorant product)

The viscosity η of the deodorant composition according to the present invention at 25°C is not limited to particular values, is preferably 10 mPa·s or more and more preferably 50 mPa·s or more, and is preferably 5,000 mPa·s or less and more preferably 3,000 mPa·s or less in order to easily discharge the deodorant composition from a container and to improve the coating properties.

The viscosity η is determined by any methods and can be determined by using a Brookfield viscometer, for example. As for the measurement conditions, the viscosity is determined with an L-adaptor in conditions at 25°C and 12 rpm, with a rotor No. 2 in conditions at 25°C and 30 rpm, or with a rotor No. 3 in conditions at 25°C and 30 rpm, for example. As the Brookfield viscometer, "TV-20" manufactured by Toki Sangyo Co., Ltd. is usable.

### (Deodorant product)

A deodorant product according to the present invention comprises a container and the deodorant composition filled in the container.

The container is exemplified by roll-on containers, spray containers (aerosol containers and non-aerosol containers), and tube containers. The container is preferably a roll-on container. The roll-on deodorant product eliminates the necessity of once applying a composition onto a hand and then applying the composition onto an application object, and enables direct and uniform application of the composition onto the application object. In other words, the roll-on container has excellent coating properties. For example, it is easy to directly apply the deodorant composition in the roll-on container onto the skin such as the underarm. In addition, the roll-on deodorant product is more likely to achieve the effect of improving the remaining performance of the bactericide after application and the durability of the bactericidal effect after application. The roll-on container comprises a roll at an application moiety. The roll may be a cylindrical member or a spherical member.

The deodorant composition according to the present invention is preferably used to be applied to the skin and is preferably used to be applied to the underarm. The deodorant composition according to the present invention is preferably a deodorant composition for skin and is preferably a deodorant composition for underarm. The deodorant composition according to the present invention can be used to be applied to the skin except the underarm.

The deodorant product according to the present invention is preferably used in such a way that the composition is applied to the skin, and is preferably used in such a way that the composition is applied to the underarm. The deodorant product according to the present invention is preferably a deodorant product for skin and is preferably a deodorant product for underarm.

The deodorant product according to the present invention may be an aerosol preparation or a non-aerosol preparation. The deodorant product according to the present invention is preferably a non-aerosol preparation because the effect of improving the remaining performance of the bactericide after application and the durability of the bactericidal effect after application is more likely to be achieved.

The dosage form of the deodorant product according to the present invention is not limited to particular forms and is exemplified by a roll-on form, a mist form, a stick form, a cream form, a gel form, and an aerosol spray form. Among them, a roll-on form or a mist form is preferred.

### Examples

The present invention will next be described in detail with reference to examples. The present invention is not limited to the following examples. The units of the amounts are "% by mass" unless otherwise specified.

In Examples and Comparative Examples, the following components were used.

(Ethanol (A))
   Ethanol
(Bactericide (B))
   Isopropylmethylphenol
   Benzalkonium chloride
(Hydrophobized hydroxyalkyl cellulose (C))
   Hydroxypropyl methylcellulose stearoxy ether ("SANGELOSE 60M" manufactured by Daido Chemical Corporation)
(Ester compound (D))
   Glyceryl tri-2-ethylhexanoate (a molecular weight of 470, a logP value of 8.919)
   Diisopropyl adipate (a molecular weight of 230, a logP value of 2.676)
   Diisobutyl adipate (a molecular weight of 258, a logP value of 3.17)
   Polypropylene glycol-3 benzyl ether myristate (a molecular weight of 548)
   Isononyl isononanoate (a molecular weight of 284, a logP value of 7.098)
   Isopropyl myristate (a molecular weight of 270, a logP value of 7.43 ± 0.21)
   Propylene glycol isostearate (a molecular weight of 342, a logP value of 7.826)
   Isopropyl palmitate (a molecular weight of 298, a logP value of 8.49 ± 0.21)
   2-Ethylhexyl palmitate (a molecular weight of 368, a logP value of 8.93)
   Pentaerythrityl tetraoctanoate (a molecular weight of 640, a logP value of 12.076)
   Octyldodecyl myristate (a molecular weight of 508, a logP value of 15.914)
(Nonionic surfactant (E))
   Polyoxyethylene polyoxypropylene decyl tetradecyl ether ("PEN-4620" manufactured by Nihon Surfactant Kogyo K.K.)
(Antiperspirant (F))
   Aluminum hydroxychloride
(Water)
   Purified water
(Other components)
   Hydroxypropylcellulose
   Polyethylene glycol 400

### (Examples 1 to 11 and Comparative Examples 1 to 3)

The components shown in Table 1 were mixed in the corresponding amounts (contents, the unit was % by mass) as shown in Table 1 and blended with a disperser to give deodorant compositions. Each deodorant composition obtained had a viscosity η of not less than 10 mPa·s and not more than 3,000 mPa·s.

The obtained deodorant composition was filled in a roll-on container to give a deodorant product.

### (Evaluation of residual ratio of bactericide)

An artificial sweat was prepared in accordance with JIS L0848: 2004. Specifically, 0.5 g of L-histidine hydrochloride monohydrate, 5 g of sodium chloride, and 2.2 g of sodium dihydrogen phosphate dihydrate were dissolved in water, next 15 mL of 0.1 mol/L sodium hydroxide was added, and water was added so as to give a total volume of 1 L to prepare an acidic artificial sweat. The obtained acidic artificial sweat had a pH of 5.5.

To a keratin fabric, 20 µL of the obtained deodorant composition was applied. In a 100-mL beaker, 20 mL of the acidic artificial sweat was placed. The keratin fabric after the application was placed in the beaker, then the whole was shaken at 100 rpm for 10 minutes, and the keratin fabric after shaking was obtained.

In a 15-mL centrifuge tube, 10 mL of methanol was placed, and the keratin fabric after shaking was placed in the centrifuge tube. The whole was shaken at 150 rpm for 10 minutes. The liquid in the centrifuge tube was filtered through a 0.45-µm membrane-filter.

The amount (Y) of the bactericide in the filtrate was determined by HPLC. Separately, 20 µL of the obtained deodorant composition was applied to a keratin fabric to prepare a test sample, then the test sample was treated in the same manner as the above except that the sample was not shaken in an acidic artificial sweat, and the amount (X) of the bactericide in the filtrate was determined. The amount (X) of the bactericide was used as the standard, and the residual ratio ((Y)/(X) × 100%) of the bactericide was calculated.

Table 1 shows the formulations and evaluation results.

**[Table 1]**

| | | Example 1 | Example 2 | Example 3 | Example 4 | Example 5 | Example 6 | Example 7 | Example 8 | Example 9 | Example 10 | Example 11 | Comparative Example 1 | Comparative Example 2 | Comparative Example 3 |
|---|---|---|---|---|---|---|---|---|---|---|---|---|---|---|---|
| Component (A) | Ethanol | 70.0 | 70.0 | 70.0 | 70.0 | 70.0 | 70.0 | 70.0 | 70.0 | 70.0 | 70.0 | 70.0 | 70.0 | 70.0 | 70.0 |
| Component (B) | Isopropylmethylphenol | 0.10 | 0.10 | 0.10 | 0.10 | 0.10 | 0.10 | 0.10 | 0.10 | 0.10 | 0.10 | 0.10 | 0.10 | 0.10 | 0.10 |
| Component (C) | Hydroxypropyl methylcellulose stearoxy ether | 0.80 | 0.80 | 0.80 | 0.80 | 0.80 | 0.80 | 0.80 | 0.80 | 0.80 | 0.80 | 0.80 | | | 0.80 |
| Component (D) | Glyceryl tri-2-ethylhexanoate | 1.0 | | | | | | | | | | | | 1.0 | |
| | Diisopropyl adipate | | 1.0 | | | | | | | | | | | | |
| | Diisobutyl adipate | | | 1.0 | | | | | | | | | | | |
| | Polypropylene glycol-3 benzyl ether myristate | | | | 1.0 | | | | | | | | | | |
| | Isononyl isononanoate | | | | | 1.0 | | | | | | | | | |
| | Isopropyl myristate | | | | | | 1.0 | | | | | | | | |
| | Propylene glycol isostearate | | | | | | | 1.0 | | | | | | | |
| | Isopropyl palmitate | | | | | | | | 1.0 | | | | | | |
| | 2-Ethylhexyl palmitate | | | | | | | | | 1.0 | | | | | |
| | Pentaerythrityl tetraoctanoate | | | | | | | | | | 1.0 | | | | |
| | Octyldodecyl myristate | | | | | | | | | | | 1.0 | | | |
| Component (E) | Polyoxyethylene polyoxypropylene decyl tetradecyl ether | 1.0 | 1.0 | 1.0 | 1.0 | 1.0 | 1.0 | 1.0 | 1.0 | 1.0 | 1.0 | 1.0 | 0.30 | 0.30 | 1.0 |
| Component (F) | Aluminum hydroxychloride | 3.0 | 3.0 | 3.0 | 3.0 | 3.0 | 3.0 | 3.0 | 3.0 | 3.0 | 3.0 | 3.0 | 3.0 | 3.0 | 3.0 |
| Others | Hydroxypropylcellulose | 0.10 | 0.10 | 0.10 | 0.10 | 0.10 | 0.10 | 0.10 | 0.10 | 0.10 | 0.10 | 0.10 | 0.10 | 0.10 | 0.10 |
| | Purified water | Remainder | Remainder | Remainder | Remainder | Remainder | Remainder | Remainder | Remainder | Remainder | Remainder | Remainder | Remainder | Remainder | Remainder |
| Total | | 100 | 100 | 100 | 100 | 100 | 100 | 100 | 100 | 100 | 100 | 100 | 100 | 100 | 100 |
| Residual ratio of bactericide (IPMP) (%) | | 65.8 | 71.4 | 62.8 | 72.9 | 66.7 | 68.4 | 74.8 | 70.8 | 61.3 | 61.9 | 66.9 | 39.3 | 49.7 | 59.5 |

### (Example 12 and Comparative Example 4)

The components shown in Table 2 were mixed in the corresponding amounts (contents, the unit was % by mass) as shown in Table 2 and blended with a disperser to give deodorant compositions. Each deodorant composition obtained had a viscosity η of not less than 10 mPa·s and not more than 3,000 mPa·s.

**[Table 2]**

| | | Example 12 | Comparative Example 4 |
|---|---|---|---|
| Component (A) | Ethanol | 70 | 75 |
| Component (B) | Isopropylmethylphenol | 0.1 | 0.1 |
| | Benzalkonium chloride | 0.1 | 0.1 |
| Component (C) | Hydroxypropyl methylcellulose stearoxy ether | 0.8 | - |
| Component (D) | Glyceryl tri-2-ethylhexanoate | 2.0 | - |
| | Isononyl isononanoate | - | 1.0 |
| Component (E) | Polyoxyethylene polyoxypropylene decyl tetradecyl ether | 1.0 | - |
| Component (F) | Aluminum hydroxychloride | 5.0 | 5.0 |
| Others | Hydroxypropylcellulose | 0.3 | 0.3 |
| | Polyethylene glycol 400 | 2.0 | - |
| | Purified water | Remainder | Remainder |
| Total | | 100 | 100 |

### (Evaluation of remaining performance of bactericide)

Evaluation was carried out by three male subjects.

About 0.2 g of deodorant composition of Example 12 was applied onto one underarm of each subject, and about 0.2 g of deodorant composition of Comparative Example 4 was applied onto the other underarm.

After 8 hours of the application, each underarm was wiped with a fabric impregnated with 99% ethanol three times. Next, the bactericide components (isopropylmethylphenol and benzalkonium chloride) in each fabric were extracted with 60% methanol, and the extraction amounts were analyzed by high performance liquid chromatography (HPLC).

The mean value of the extraction amounts of isopropylmethylphenol obtained from the underarms to which the deodorant composition of Example 12 had been applied of all the subjects was regarded as (I1), whereas the mean value of the extraction amounts of isopropylmethylphenol obtained from the underarms to which the deodorant composition of Comparative Example 4 had been applied of all the subject was regarded as (I2). The mean value of the extraction amounts of benzalkonium chloride obtained from the underarms to which the deodorant composition of Example 12 had been applied of all the subjects was regarded as (B1), whereas the mean value of the extraction amounts of benzalkonium chloride obtained from the underarms to which the deodorant composition of Comparative Example 4 had been applied of all the subject was regarded as (B2).

The value obtained by dividing (I1) by (I2), [(I1)/(I2)], was about 3.3, and the value obtained by dividing (B1) by (B2), [(B1)/(B2)], was about 1.5. In other words, the remaining amount of isopropylmethylphenol of the deodorant composition of Example 12 is about 3.3 times larger than that of the deodorant composition of Comparative Example 4 after 8 hours of the application, and the remaining amount of benzalkonium chloride of the deodorant composition of Example 12 is about 1.5 times larger than that of the deodorant composition of Comparative Example 4. The result indicated that the deodorant composition of Example 12 had excellent remaining performance of the bactericides in the composition.

### (Evaluation of durability of deodorizing effect)

Evaluation was carried out by seven male subjects. The body of each subject was washed, and then the evaluation was carried out.

About 0.2 g of deodorant composition of Example 12 was applied onto one underarm (called "underarm of the applied side") of each subject, and none was applied onto the other underarm (called "underarm of the non-applied side").

After 24 hours of the application, the odors of the underarms (the underarm of the applied side and the underarm of the non-applied side) of the respective subjects were evaluated in accordance with the following evaluation criteria. Five specialists evaluated the odor.

### <Evaluation criteria>

Score 0: odorless
Score 1: barely detectable odor
Score 2: distinguishable odor
Score 3: easily detectable odor
Score 4: strong odor
Score 5: intense odor

As a result, the underarms of the applied side had significantly weaker odor (smaller score) than that of the underarms of the non-applied side by Wilcoxon signed rank sum test. In other words, the deodorizing effect of the deodorant composition of Example 12 continued even after 24 hours. The result indicated that the deodorant composition of Example 12 had excellent durability of the deodorizing effect.

### (Evaluation of durability of bactericidal effect)

Evaluation was carried out by seven male subjects. The body of each subject was washed, and the evaluation was carried out.

About 0.2 g of the deodorant composition of Example 12 was applied onto one underarm (called "underarm of the applied side") of each subject, and none was applied onto the other underarm (called "underarm of the non-applied side").

After 24 hours of the application, the underarms of the applied side were compared with the underarms of the non-applied side by ATP (adenosine triphosphate) swab test.

### <ATP swab test>

After 24 hours of the application, the underarms were wiped with a cotton swab to collect bacteria. The bacteria collected from the underarm of the applied side were extracted with a physiological saline, and the physiological saline was regarded as a "sample of the applied side". The bacteria collected from the underarm of the non-applied side were extracted with a physiological saline, and the physiological saline was regarded as a "sample of the non-applied side". To each of the sample of the applied side and the sample of the non-applied side, an ATP extraction reagent and an ATP luminescence reagent were added, and the amount of luminescence was determined with an ATP measuring instrument ("LUMITESTER C-110" manufactured by Kikkoman Biochemifa Company).

As a result, the amount of ATP luminescence of the sample of the applied side was about 400 (unit: RLU), whereas the amount of ATP luminescence of the sample of the non-applied side was about 18,200 (unit: RLU). Here, a larger amount of ATP luminescence means a larger number of bacteria.

In other words, the bactericidal effect of the deodorant composition of Example 12 continued even after 24 hours. The result indicated that the deodorant composition of Example 12 had excellent durability of the bactericidal effect.

### (Examples 13 to 21)

The components shown in Table 3 were mixed in the corresponding amounts (contents, the unit was % by mass) as shown in Table 3 and blended with a disperser to give deodorant compositions. Each deodorant composition obtained had a viscosity η of not less than 10 mPa·s and not more than 3,000 mPa·s.

**[Table 3]**

| | | Example 13 | Example 14 | Example 15 | Example 16 | Example 17 | Example 18 | Example 19 | Example 20 | Example 21 |
|---|---|---|---|---|---|---|---|---|---|---|
| Component (A) | Ethanol | 60.0 | 60.0 | 60.0 | 60.0 | 60.0 | 60.0 | 60.0 | 60.0 | 60.0 |
| Component (B) | Isopropylmethylphenol | 0.10 | 0.10 | 0.10 | 0.10 | 0.10 | 0.10 | 0.10 | 0.10 | 0.10 |
| | Benzalkonium chloride | 0.10 | 0.10 | 0.10 | 0.10 | 0.10 | 0.10 | 0.10 | 0.10 | 0.10 |
| Component (C) | Hydroxypropyl methylcellulose stearoxy ether | 0.80 | 3.00 | 0.50 | 0.20 | 0.30 | 0.50 | 0.65 | 0.80 | 0.80 |
| Component (D) | Glyceryl tri-2-ethylhexanoate | 1.0 | 5.0 | 0.3 | 1.0 | 1.0 | 1.0 | 1.0 | 0.3 | 0.7 |
| Component (E) | Polyoxyethylene polyoxypropylene decyl tetradecyl ether | 1.0 | 1.0 | 1.0 | 1.0 | 1.0 | 1.0 | 1.0 | 1.0 | 1.0 |
| Component (F) | Aluminum hydroxychloride | 3.0 | 3.0 | 3.0 | 3.0 | 3.0 | 3.0 | 3.0 | 3.0 | 3.0 |
| Others | Hydroxypropylcellulose | 0.10 | 0.10 | 0.10 | 0.10 | 0.10 | 0.10 | 0.10 | 0.10 | 0.10 |
| | Purified water | Remainder | Remainder | Remainder | Remainder | Remainder | Remainder | Remainder | Remainder | Remainder |
| Total | | 100 | 100 | 100 | 100 | 100 | 100 | 100 | 100 | 100 |

The further formulation examples are shown below.

**(Formulation Example 1) Deodorant mist**

| | |
|---|---|
| Ethanol | 60.0% by mass |
| Isopropylmethylphenol | 0.1% by mass |
| Triclosan | 0.2% by mass |
| Hydroxypropyl methylcellulose stearoxy ether | 0.8% by mass |
| Glyceryl tri-2-ethylhexanoate | 2.0% by mass |
| Polyoxyethylene polyoxypropylene decyl tetradecyl ether | 1.0% by |
| mass | |
| Zinc p-phenolsulfonate | 0.3% by mass |
| Hydroxypropylcellulose | 0.3% by mass |
| Polyethylene glycol 400 | 2.0% by mass |
| l-Menthol | 0.4% by mass |
| Purified water | remainder |
| Total amount | 100.0% by mass |

**(Formulation Example 2) Deodorant mist**

| | |
|---|---|
| Ethanol | 60.0% by mass |
| Benzalkonium chloride | 0.1% by mass |
| Triclosan | 0.2% by mass |
| Hydroxypropyl methylcellulose stearoxy ether | 0.8% by mass |
| Glyceryl tri-2-ethylhexanoate | 2.0% by mass |
| Polyoxyethylene polyoxypropylene decyl tetradecyl ether | 1.0% by |
| mass | |
| Zinc p-phenolsulfonate | 0.3% by mass |
| Hydroxypropylcellulose | 0.3% by mass |
| Polyethylene glycol 400 | 2.0% by mass |
| l-Menthol | 0.3% by mass |
| l-Menthyl glyceryl ether | 0.2% by mass |
| Plant extract | 0.1% by mass |
| Purified water | remainder |
| Total amount | 100.0% by mass |

**(Formulation Example 3) Deodorant lotion**

| | |
|---|---|
| Ethanol | 50.0% by mass |
| Isopropylmethylphenol | 0.1% by mass |
| Benzalkonium chloride | 0.1% by mass |
| Hydroxypropyl methylcellulose stearoxy ether | 0.8% by mass |
| Glyceryl tri-2-ethylhexanoate | 2.0% by mass |
| Polyoxyethylene polyoxypropylene decyl tetradecyl ether | 1.0% by |
| mass | |
| Aluminum hydroxychloride | 5.0% by mass |
| Hydroxypropylcellulose | 0.3% by mass |
| Polyethylene glycol 400 | 2.0% by mass |
| Crosslinked polystyrene | 2.0% by mass |
| Purified water | remainder |
| Total amount | 100.0% by mass |

**(Formulation Example 4) Deodorant lotion**

| | |
|---|---|
| Ethanol | 50.0% by mass |
| Isopropylmethylphenol | 0.1% by mass |
| Benzalkonium chloride | 0.1% by mass |
| Hydroxypropyl methylcellulose stearoxy ether | 0.2% by mass |
| Glyceryl tri-2-ethylhexanoate | 1.0% by mass |
| Polyoxyethylene polyoxypropylene decyl tetradecyl ether | 1.0% by |
| mass | |
| Zinc p-phenolsulfonate | 0.3% by mass |
| Hydroxypropylcellulose | 0.1% by mass |
| l-Menthol | 0.5% by mass |
| l-Menthyl glyceryl ether | 0.1% by mass |
| Perfume | 0.3% by mass |
| Plant extract | 0.1% by mass |
| Purified water | remainder |
| Total amount | 100.0% by mass |

**(Formulation Example 5) Deodorant lotion**

| | |
|---|---|
| Ethanol | 50.0% by mass |
| Isopropylmethylphenol | 0.1% by mass |
| Benzalkonium chloride | 0.1% by mass |
| Hydroxypropyl methylcellulose stearoxy ether | 0.2% by mass |
| Glyceryl tri-2-ethylhexanoate | 1.0% by mass |
| Polyoxyethylene polyoxypropylene decyl tetradecyl ether | 1.0% by |
| mass | |
| Zinc p-phenolsulfonate | 0.3% by mass |
| Hydroxypropylcellulose | 0.1% by mass |
| Nylon powder | 1.0% by mass |
| Crosslinked polystyrene | 1.0% by mass |
| l-Menthol | 0.2% by mass |
| l-Menthyl glyceryl ether | 0.1% by mass |
| Perfume | 0.2% by mass |
| Purified water | remainder |
| Total amount | 100.0% by mass |

## Claims

1. A deodorant composition comprising:
ethanol;
a bactericide;
a hydrophobized hydroxyalkyl cellulose; and
an ester compound having a molecular weight of 150 or more.

2. The deodorant composition according to claim 1, wherein the ester compound has a logP value of not less than 1 and not more than 40.

3. The deodorant composition according to claim 1, wherein the ester compound is an ester compound selected from the group consisting of isononyl isononanoate, glyceryl tri-2-ethylhexanoate, diisopropyl adipate, diisobutyl adipate, polypropylene glycol-3 benzyl ether myristate, isopropyl myristate, propylene glycol isostearate, isopropyl palmitate, 2-ethylhexyl palmitate, cetyl 2-ethylhexanoate, pentaerythrityl tetraoctanoate, octyldodecyl myristate, and pentaerythrityl tetraisostearate.

4. The deodorant composition according to any one of claims 1 to 3, wherein the bactericide is a bactericide selected from the group consisting of isopropylmethylphenol, benzalkonium chloride, hinokitiol, triclosan, and salicylic acid.

5. The deodorant composition according to any one of claims 1 to 4, further comprising a nonionic surfactant.

6. The deodorant composition according to any one of claims 1 to 5, further comprising an antiperspirant.

7. The deodorant composition according to claim 6, wherein the antiperspirant is an antiperspirant selected from the group consisting of aluminum hydroxychloride, zinc p-phenolsulfonate, and alums.

8. A deodorant product comprising:
a container; and
the deodorant composition according to any one of claims 1 to 7, the deodorant composition being filled in the container.

9. The deodorant product according to claim 8, wherein the deodorant product is a non-aerosol preparation.
